# EUROPEAN PATENT APPLICATION

(11) **EP 1 588 616 A2**
(43) Date of publication of application: **26.10.2005**
(21) Application number: 05103060.9
(22) Date of filing: 18.04.2005
(51) Int. Cl.: A01N 1/02

(54) **Method of transporting transgenic Xenopus laevis oocytes**

(30) Priority: 19.04.2004 US 562981 P
(71) Applicant: Becton Dickinson and Company, Franklin Lakes, New Jersey 07417 (US)
(72) Inventor: XIAO, Guangqing, Shrewsbury, Massachusetts 01545 (US)
(74) Representative: von Kreisler Selting Werner

(57) **Abstract**

A method is disclosed herein of providing transgenic *Xenopus laevis* oocytes. The method comprises preparing transgenic *Xenopus laevis* oocytes at a first location and transporting the transgenic *Xenopus laevis* oocytes to a second location remote from the first location. Preferably, the transgenic *Xenopus laevis* oocytes are prepared by injecting *Xenopus laevis* oocytes with a cRNA or cDNA encoding various human or animal membrane proteins. In a further aspect of the subject invention, a culture system is provided which comprises at least one vessel and at least one transgenic *Xenopus laevis* oocyte disposed in the vessel. The vessel is sealed so as to allow for transportation of the vessel. Advantageously, with the subject invention, a user is able to conduct testing, e.g., drug transport assays, with transgenic *Xenopus laevis* oocytes without the arduous task of injecting *Xenopus laevis* oocytes with cRNA or cDNA encoding various human or animal membrane proteins.

## Description

### Cross-Reference to Related Application

This Application claims priority to U.S. Provisional Application No. 60/562,981, filed April 19, 2004, the entire contents of which are incorporated by reference herein.

### Field of the Invention

The present invention relates to a method of providing *Xenopus laevis* oocytes which have been pre-injected with a cRNA or cDNA encoding human or animal membrane proteins, such as membrane transport proteins.

### Background of the Invention

The oocyte from the South African clawed *Xenopus laevis* frog is an often used functional expression system. Oocyte expression systems have been used to study the function of membrane proteins such as transporters, ion channels, and pumps. The oocyte expression systems demonstrate low backgrounds, high expression levels and proper post-translational modifications.

Setting up drug transport assays for various human and animal membrane transport proteins (also referred to herein as transporters), such as human Organic Cation Transporter I (hOCT1, *SLC 22A1*), human Organic Anion Transporting Polypeptide I (hOATP1, *SLC21A3*), human Organic Anion Transporting Polypeptide 2 (hOATP2, *SLC21A6*), human Organic Anion Transporting Polypeptide 8 (hOATP8, *SLC21A8*), human Na⁺-Taurocholate Cotransport Protein (hNTCP, *SLC10A1*), rat Organic Anion Transporting Polypeptide (rOatp1, *Slc21a1*), human Peptide Transporter I (hPEPT1, *SLC15A1*), human Peptide Transporter 2 (hPEPT2, *SLC15A2*), human Organic Anion Transporter 1 (hOAT1, *SLC22A6*), human Organic Anion Transporter 3 (hOAT3, *SLC22A8*), rat Organic Anion Transporter 3 (rOat3, *Slc22a8*), and rat Organic Anion Transporting Polypeptide 4 (rOatp4, *Slc21a10*) can present many technical challenges. Expressing these transporters in oocytes for their function characterizations is difficult and time consuming owing to the scale of the process.

### Summary of the Invention

A method is disclosed herein ofproviding transgenic *Xenopus laevis* oocytes. The method comprises preparing transgenic *Xenopus laevis* oocytes at a first location and transporting the transgenic *Xenopus laevis* oocytes to a second location remote from the first location. Preferably, the transgenic *Xenopus laevis* oocytes are prepared by injecting *Xenopus laevis* oocytes with a cRNA or cDNA encoding various human or animal membrane proteins.

In a further aspect of the subject invention, a culture system is provided which comprises at least one vessel and at least one transgenic *Xenopus laevis* oocyte disposed in the vessel. The vessel is sealed so as to allow for transportation of the vessel. Advantageously, with the subject invention, a user is able to conduct testing, e.g., drug transport assays, with transgenic *Xenopus laevis* oocytes without the arduous task of injecting *Xenopus laevis* oocytes with cRNA or cDNA encoding various human or animal membrane proteins.

The cRNA or cDNA may encode various human or animal membrane proteins, such as human or animal membrane transport proteins selected from human Organic Cation Transporter 1 (hOCT1), human Organic Anion Transporting Polypeptide 1 (hOATP1), human Organic Anion Transporting Polypeptide 2 (hOATP2), human Organic Anion Transporting Polypeptide 8 (hOATP8), human Na⁺-Taurocholate Cotransport Protein (hNTCP), rat Organic Anion Transporting Polypeptide (rOatp1), human Peptide Transporter 1 (hPEPT1), human Peptide Transporter 2 (hPEPT2), human Organic Anion Transporter 1 (hOAT1), human Organic Anion Transporter 3 (hOAT3), rat Organic Anion Transporter 3 (rOat3), and rat Organic Anion Transporting Polypeptide 4 (rOatp4).

As used herein, "transgenic" refers to oocytes that express a human or animal membrane protein, such as a membrane transport protein, in addition to the normal complement of proteins.

These and other features of the invention will be better understood through a study of the following detailed description and accompanying drawings.

### Brief Description of the Figures

FIG. I is a flowchart representing the method of the subject invention.

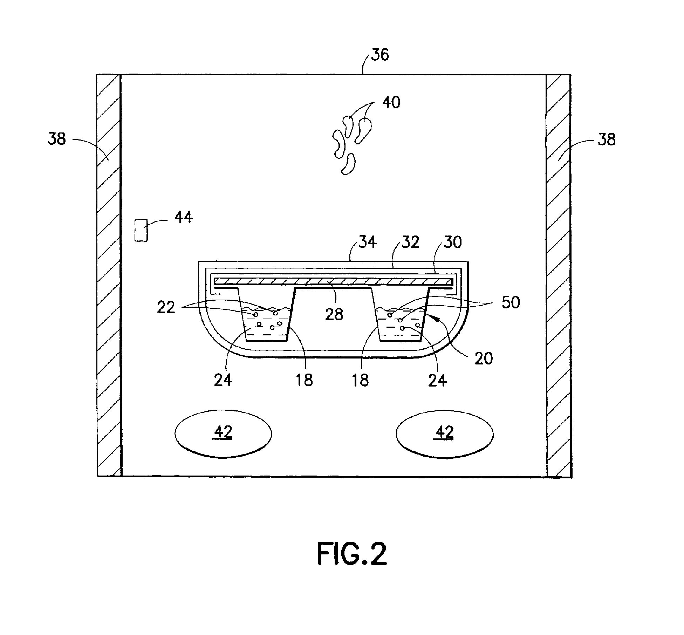

FIG. 2 is a cross-section of packaged transgenic and control *Xenopus laevis* oocytes.

### Detailed Description of the Invention

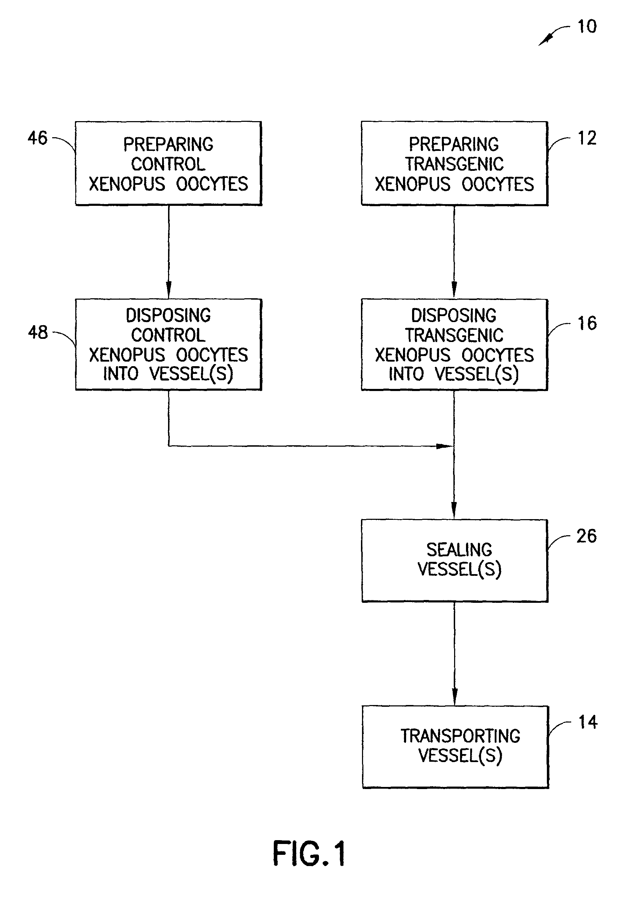

With reference to FIG. 1, a method 10 is set forth ofproviding transgenic *Xenopus laevis* oocytes.

In an initial step **12,** the transgenic *Xenopus laevis* oocytes are prepared. Any known technique can be used to prepare the transgenic *Xenopus laevis* oocytes. Preferably, the transgenic *Xenopus laevis* oocytes are prepared by injecting *Xenopus laevis* oocytes with cRNA or cDNA encoding a human or animal membrane protein, such as a membrane transport protein. The cRNA or cDNA may encode various human or animal membrane proteins, including membrane transport proteins. By way of nonlimiting example, the cRNA or cDNA may encode any of the following animal or human membrane transport proteins: human Organic Cation Transporter 1 (hOCT1, *SLC 22A1*), human Organic Anion Transporting Polypeptide 1 (hOATP1, *SLC21A3*), human Organic Anion Transporting Polypeptide 2 (hOATP2, *SLC21A6*), human Organic Anion Transporting Polypeptide 8 (hOATP8, *SLC21A8*), human Na⁺-Taurocholate Cotransport Protein (hNTCP, *SLC10A1*), rat Organic Anion Transporting Polypeptide (rOatp1, *Slc21a1*), human Peptide Transporter 1 (hPEPT1, *SLC15A1*), human Peptide Transporter 2 (hPEPT2, *SLC15A2*), human Organic Anion Transporter I (hOAT1, *SLC22A6*), human Organic Anion Transporter 3 (hOAT3, *SLC22A8*), rat Organic Anion Transporter 3 (rOat3, *Slc22a8*), and rat Organic Anion Transporting Polypeptide 4 (rOatp4, *Slc21a10*). These proteins and the cRNA's and the cDNA's that encode them are known in the art.

In a preferred embodiment, *Xenopus laevis* frogs or *Xenopus laevis* oocytes may be obtained from NASCO, Ft. Atkinson, WI 53538. The cRNA or cDNA encoding human or animal membrane proteins may be injected by standard techniques. See, Wagner, et al., *Cellular Physiol. Biochem.,* 2000, 10, 1-12. Also, conventional molecular biological or cell biological techniques that can be employed with the present invention are disclosed in *Current Protocols in Molecular Biology,* Volumes I-III (F. Ausubel, ed. 1994).

Once the transgenic *Xenopus laevis* oocytes are prepared, the transgenic *Xenopus laevis* oocytes can be transported from the site of preparation to a remote site, as represented by step **14.** With the subject invention, the transgenic *Xenopus laevis* oocytes are prepared at a first location (e.g., a manufacturer's facility) and transported to a remote, second location (e.g., the customer's facility). Any mode of transportation can be used, including, but not limited to automobile, truck, airplane, train, or ship transport, or any combinations thereof. It is preferred that the transgenic *Xenopus laevis* oocytes be transported for a period of no more than 2-4 days. The viability of transgenic *Xenopus laevis* oocytes is typically a period of 7 days.

Preferably, the transgenic *Xenopus laevis* oocytes are disposed in one or more vessels (step **16**) prior to being transporting. The vessels may be of any known configuration, such as test tubes, vials, flasks, etc. With reference to FIG. 2, vessels **18** are preferably wells of a multiwell plate **20.** It is further preferred that the multiwell plate **20** conform to conventional multiwell plate standards (e.g., the Standards of the Society of Biomolecular Screening) so as to be usable in drug assay handling equipment (e.g., high throughput screening (HTS) equipment). The multiwell plate **20** is preferably the multiwell plate commercially referred to under the trademark "BD Falcon™ Flip-Lock Packaging" from Becton Dickinson & Co., Franklin Lakes, New Jersey. This specific multiwell plate includes an array of twelve wells, although any number of wells can be used with the subject invention.

With further reference to FIG. 2, any number of transgenic *Xenopus laevis* oocytes **22** may be disposed into the vessels **18.** It is preferred that 4-5 transgenic *Xenopus laevis* oocytes **22** be disposed per each of the vessels **18.**

A buffer solution **24** is also preferably disposed into the vessels **18** to suspend the transgenic *Xenopus laevis* oocytes **22.** The buffer solution **24** may be any solution which will maintain the viability of the transgenic *Xenopus laevis* oocytes **22** for an extended period to allow for transportation to the remote site. The buffer solution **24** may be ND96 (96 mM NaCl, 2 mM KCl, 1 mM CL₂, 1.8 mM CaCl₂, 50 µg/ml Gentamicin, pH 7.4) or Modified Barth Medium (88 mM NaCl, 0.82 mM MgSO₄, 0.41 mM CaCl₂, 0.33 mM Ca(NO₃)₂, 2.4 mM NaHCO₃, 10 mM HEPES, 50 µg/ml Gentamicin). It is preferred that approximately 5 ml of the buffer solution **24** be provided to suspend 4-5 transgenic *Xenopus laevis* oocytes in each one of the vessels **18.** It is further preferred that with the vessels **18** being part of a unitary structure (e.g., wells of a single multiwell plate), each of the vessels **18** of the unitary structure include an equal amount of the buffer solution **24,** even in the vessels 18 where no transgenic *Xenopus laevis* oocytes **22** may be present. Equal amounts of the buffer solution **24** will reduce sloshing effects during transportation and reduce potential damage to the transgenic *Xenopus laevis* oocytes **22.**

The buffer solution **24** may be disposed into the vessels **18** using any known technique. It is preferred to dispose the buffer solution **24** into the vessels **18** prior to disposing the transgenic *Xenopus laevis* oocytes **22.** The transgenic *Xenopus laevis* oocytes **22** may be disposed into the vessels **18** using any known technique, such as transfer pipette.

It is also preferred that the vessels be sealed prior to transportation, as indicated by step **26** in FIG. 1. The vessels are preferably sealed liquid-tight to prevent leakage of the transgenic *Xenopus laevis* oocytes and the buffer solution. Any known sealing arrangement can be used. For illustrative purposes, and with reference to FIG. 2, a resilient gasket or seal member **28** (e.g., a silicone gasket) may be disposed across the vessel **18** to provide a liquid-tight barrier against leakage. The seal member **28** may be fixed relative to the vessel **18** using any known technique. For example, a lid **30** may be placed over the seal member **28** which is threaded, hinged, latched or otherwise removably fixed relative to the vessel **18** (e.g., being latched onto the multiwell plate **20**). The lid **30** may provide a backing force against the seal member **28** to enhance the seal provided thereby. The seal member **28** may also be a foil or plastic film removably fixed relative to the vessel (e.g., by heat bonding).

Transgenic *Xenopus laevis* oocytes are temperature sensitive, and it is preferred to maintain the temperature of the transgenic *Xenopus laevis* oocytcs during the transportation step **14.** More particularly, it is preferred that the temperature be maintained in the range of 10-20 °C, and, more preferably, at a temperature of 16 °C. The temperature can be maintained by any known technique. With the subject invention, the transgenic *Xenopus laevis* oocytes may be initially chilled to the desired temperature and packaged in insulative material to maintain the chilled temperature. For example, with reference to FIG. 2, a paper sleeve or envelope **32** (e.g., heavy weight paper or paperboard) and/or a plastic sleeve or pouch **34** may be placed about the vessel **18** to provide insulation. The paper sleeve or envelope **32** and/or the plastic sleeve or pouch **34** may be tightly wrapped about the vessel **18** to additionally provide backing force to the seal member **28.** In addition, external insulative packaging may be used. With reference to FIG. 2, the vessel **18** may be packed into a carton **36** having an insulative liner **38.** Loose packaging material **40** (e.g., foam segments) may also be provided to add not only insulation, but also shock-resistance. Further, pre-cooled media **42** may be packaged externally of the transgenic *Xenopus laevis* oocytes (e.g., pre-cooled gel packs chilled to 4 °C) to further maintain the desired temperature. To allow for inspection of temperature fluctuations during transportation, a thermal digital recorder **44** may be packaged with the transgenic *Xenopus laevis* oocytes.

As will be appreciated by those skilled in the art, the transgenic *Xenopus laevis* oocytes of the subject invention can be used as part of a culture system. In addition to the transgenic *Xenopus laevis* oocytes being sealed in one or more vessels for transportation, the culture system may include control *Xenopus laevis* oocytes. With reference to FIG. 1, the control *Xenopus laevis* oocytes may be prepared by providing uninjected *Xenopus laevis* oocytes (i.e., not injected with a cRNA or cDNA encoding a human or animal membrane protein) or by injecting water into the *Xenopus laevis* oocytes in vivo (step **46**). Thereafter, the control *Xenopus laevis* oocytes may be disposed into one or more vessels (step **48**), sealed (step **26**) and transported (step **14**), including maintaining the temperature thereof, in the same manner as described above. With reference to FIG. 2, control *Xenopus laevis* oocytes **50** are shown. It is, however, preferred that a unitary structure of vessels (e.g., a multiwell plate) not contain both transgenic *Xenopus laevis* oocytes and control *Xenopus laevis* oocytes so as to avoid confusion; it is preferred that separated vessels be used.

The culture system may also include other components, such as reagents for analysis. The reagents may include ND96 buffer solution, as described above, (e.g., 500 ml); sodium (Na⁺) buffer solution (e.g., 500 ml); and, SDS lysis buffer solution (e.g., 30 ml).

## Claims

1. A method of providing transgenic *Xenopus laevis* oocytes, the method comprising:
a) preparing transgenic *Xenopus laevis* oocytes at a first location; and,
b) transporting the transgenic *Xenopus laevis* oocytes to a second location remote from said first location.

2. The method of claim 1, wherein said preparing transgenic *Xenopus laevis* oocytes includes injecting *Xenopus laevis* oocytes with cRNA or cDNA encoding a human or animal membrane protein.

3. The method of claim 2, wherein the human or animal membrane protein is selected from the group consisting of human Organic Cation Transporter 1 (hOCT1), human Organic Anion Transporting Polypeptide I (hOATP1), human Organic Anion Transporting Polypeptide 2 (hOATP2), human Organic Anion Transporting Polypeptide 8 (hOATP8), human Na⁺-Taurocholate Cotransport Protein (hNTCP), rat Organic Anion Transporting Polypeptide (rOatp1), human Peptide Transporter 1 (hPEPT1), human Peptide Transporter 2 (hPEPT2), human Organic Anion Transporter I (hOAT1), human Organic Anion Transporter 3 (hOAT3), rat Organic Anion Transporter 3 (rOat3), and rat Organic Anion Transporting Polypeptide 4 (rOatp4).

4. The method of claim 1, further comprising disposing said transgenic *Xenopus laevis* oocytes into at least one vessel before delivering.

5. The method of claim 4, wherein said at least one vessel is a well of a multiwell plate.

6. The method of claim 5, further comprising sealing said at least one well after the disposing step but before delivering.

7. The method of claim 4, further comprising sealing said at least one vessel after the disposing step but before delivering.

8. The method of claim 4, further comprising disposing a buffer solution into said at least one vessel before delivering.

9. The method of claim 1, wherein said delivering includes maintaining the temperature of said transgenic *Xenopus laevis* oocytes.

10. The method of claim 9, wherein said temperature is maintained in the range of 10-20 °C.

11. The method of claim 10, wherein said temperature is maintained at 16 °C.

12. The method of claim 1, wherein said transporting includes transporting by a mode of transport selected from the group consisting of automobile, truck, airplane, train, ship, and combinations thereof.

13. A culture system comprising:
at least one vessel; and,
at least one transgenic *Xenopus laevis* oocyte disposed in said vessel, wherein said vessel is sealed so as to allow for transportation of said vessel.

14. The system as in claim 13, further comprising at least one *Xenopus laevis* oocyte.

15. The system as in claim 13, further comprising at least one *Xenopus laevis* oocyte pre-injected with water.

16. The system as in claim 13, further comprising a buffer solution disposed in said vessel.

17. The system as in claim 13, wherein said vessel is a well of a multiwell plate.

18. The system as in claim 13, further comprising at least one reagent.
